# EUROPEAN PATENT SPECIFICATION

(11) **EP 3 349 808 B1**
(45) Date of publication and mention of the grant of the patent: **22.07.2020**
(21) Application number: 16766020.8
(22) Date of filing: 15.09.2016
(51) Int. Cl.: C12N 5/00, A61L 27/22, A61L 27/52

(54) **IMPROVED HYDROGEL COMPOSITIONS**
VERBESSERTE HYDROGEL-ZUSAMMENSETZUNGEN
COMPOSITIONS AMÉLIORÉES POUR HYDROGELS

(30) Priority: 16.09.2015 GB 201516421
(43) Date of publication of application: 25.07.2018
(73) Proprietor: Biogelx Limited, Newhouse, Lanarkshire ML1 5UH (GB)
(72) Inventor: IRVINE, Eleanore Jane, Edinburgh Midlothian EH11 2AD (GB); HARPER, Mhairi Malone, Coatbridge North Lanarkshire ML5 3PU (GB); GOLDIE, Laura, Glasgow Lanarkshire G51 1NU (GB); CONNOLLY, Michael Leo, Bathgate West Lothian EH48 1BP (GB)
(74) Representative: Boynton, Juliette Alice
(86) International application number: PCT/EP2016/071888
(87) International publication number: WO 2017/046277

(56) References cited:
- R ORBACH ET AL.: "The rheological and structural properties of Fmoc-peptide-based hydrogels: the effect of aromatic molecular architecture on self-assembly and physical characteristics", LANGMUIR, vol. 28, 2012, pages 2015-2022, XP55313227, American Chemical Society ISSN: 0743-7463
- R ORBACH ET AL.: "Self-assembled Fmoc-peptides as a platform for the formation of nanostructures and hydrogels", BIOMACROMOLECULES, vol. 10, 2009, pages 2646-2651, XP55217882, AMERICAN CHEMICAL SOCIETY ISSN: 1525-7797
- MI ZHOU ET AL.: "Self-assembled peptide-based hydrogels as scaffolds for anchorage-dependent cells", BIOMATERIALS., vol. 30, 2009, pages 2523-2530, XP002764978, ELSEVIER SCIENCE PUBLISHERS BV., BARKING. ISSN: 0142-9612

## Description

### Introduction

The present invention relates to hydrogel compositions, including hydrogels as such, and precursors in liquid or dried form, which can act as a scaffold and support for cell growth. In particular the invention relates to hydrogel compositions which are able to self-assemble into gels and are formed from peptides and peptide derivatives.

### Background to the Invention

It is known to support cell growth on synthetic structures which resemble physiological ones, such structures including hydrogels.

Zhang et al (J. Am. Chem. Soc. 2003, 125, pp13680-13681) describes Fmoc protected dipeptides, for example Phe-Phe, Ala-Ala, Gly-Gly, Gly-Ala, Gly-Ser in combination with an aromatic stacking ligand, forming fibrous scaffolds. These were carried out at a pH of 3-5, which is too acidic for normal cell growth. No investigations were carried out at physiological pH. Other peptide-based gels are described in Gary Scott et al, Langmuir, vol. 29, 2013, pp 14321-14327; Mi Zou et al, Biomaterials, vol. 30, 2009, pp 2523-2530; and Apurba Das et al, Faraday discussions, vol. 143, 209, pp 293-303. Further peptide-based gels are described in Ron Orbach et al, Langmuir, vol. 28, 2012, pp 2015-2022 and Ron Orbach et al, Biomacromolecules, vol. 10, 2009, pp 2646-2651.

US Patent No. 8,420,605 in the name of the University of Strathcylde describes further hydrogels which comprise an aromatic stacking ligand, such as Fmoc or Cbz, together with peptides or peptide derivatives at physiological pH. A hydrogel was formed at a pH of 7 from Fmoc-Phe-Phe. Further work was carried out using other peptides, in combination with an aromatic stacking ligand, such as C-Phe-Phe, where C = Alanine, Valine, Leucine or Phenylalanine. The hydrogel formed from Fmoc-Phe-Phe was, however, too hydrophobic for attachment of cells and thus did not form a suitable scaffold for cell growth of anchorage dependant cells.

For optimising of cell growth, such hydrogels must form a sufficiently stiff gel to support cell growth and must do so under physiological pH. In addition, it is advantageous for the gel to be optically transparent to aid monitoring of the cells, not be too concentrated (as low concentration of gel components or large pore sizes between gel components can be beneficial for cell growth), and have sufficient longevity for the growth required.

It is known that use of an aromatic amino acid, for example phenylalanaine (Phe, F), in a peptide derivative, for example in combination with an aromatic stacking ligand, can act as a strong structural component which can increase gel strength through enhanced π stacking from the aromatic ring.

We have now found that combinations of peptides, in particular dipeptides, can be used to create hydrogels with tunable properties to suit conditions for growth of differing cell types.

It is an aim of the current invention to provide alternative and preferably improved hydrogels based on peptide derivatives which exhibit sufficient stiffness for and furnish a suitable environment for cell growth.

### Summary of the Invention

Accordingly, the invention provides hydrogels and precursors for hydrogels for scientific and therapeutic uses. Precursors include dry formats and pre-gelation mixtures, for example in which gelling components are provided in an aqueous format but not yet gelled. The gels and precursors are referred to as hydrogel compositions. The compositions of the invention are based on novel combinations of peptides that deliver gels that can support cell growth, especially human cell growth, with altered and preferably improved properties compared to known gels.

The invention provides for example a hydrogel composition, comprising a mixture of first and second peptide derivatives in a ratio of from 1:4 to 4:1, wherein
the first peptide derivatives comprise ASL-GA-GA,
the second peptide derivatives comprise ASL-GA-X, in which
ASL is an aromatic stacking ligand, which is Fmoc,
GA is the amino acid phenylalanine (F),
X is an amino acid selected from neutral amino acid serine (S), positively charged amino acids arginine (R), and lysine (K), and negatively charged amino acids aspartic acid (D) and glutamic acid (E).

We have found that hydrogels falling within this definition can be obtained that are sufficiently stiff to support cell growth (varying gel stiffnesses being producible to support growth of different types of cells), are clear or sufficiently clear for cells grown therein to be visualised using microscopy techniques and have sufficient longevity to support cell and tissue growth over therapeutically relevant time periods.

Many gel properties are variable according to the choice of the individual components and their concentrations and also optional additional components. The ability for the user to choose gels having predetermined stiffness will be apparent from the discussion below of the various optional and preferred features of the hydrogels of the invention. The ratio of the first and second peptide derivatives is optionally altered to alter hydrogel properties. For example, the proportion of peptides containing hydrophobic amino acids will tend to affect the overall hydrophobicity of the gel. This is one example of a property that is in use varied according to the preference of the cell type to be cultured in or on the gel. In all gels of the present invention, provision of non-hydrophobic amino acids or derivatives in the mixed gel components reduces the hydrophobicity, increasing their usefulness in supporting cell growth, an improvement over prior art gels based predominantly on Fmoc-Phe-Phe.

Specific gels made and described below have ratios of first:second peptide derivatives of approximately 1:1. In further preferred examples the ratio may be from about 2:3 to about 3:2.

Gel compositions of the invention spontaneously form 3 dimensional gels with measurable stiffness, after combination with aqueous media (optionally supplemented by other components mentioned elsewhere herein) under conditions of physiological pH, generally at pH from about 6 to about 9, more suitably at pH in the range from about pH 7 to 8, human body pH being typically about 7.4. When provided in, say, powdered form addition of a predetermined amount of known culture media containing known ions, supplements etc. yields a solution having a pH in the above range; the gel then forms and stiffens spontaneously. Alternatively, a pre-gelation mixture can be provided in liquid form; addition of a predetermined amount of media then yields a solution with pH in the gelling range above, and the gel forms.

During gel formation, fibres are formed within the gel by stacking of adjacent peptides into straight and, in some cases, branched fibres. The formation of this fibre-containing matrix is facilitated by the presence of the aromatic stacking ligands, suitably as described in US 2013/0338084 and US 8,420,605. Examples include Fmoc, Cbz and derivatives thereof. Aromatic stacking interactions are recognised and well described in the scientific literature and arise from the attractive force between the π-electron clouds in adjacent / neighbouring aromatic groups. The aromatic stacking can be affected by various factors including pH, and as described elsewhere herein the forces are strong enough so that present gels can be formed at physiological pH. In the invention, fibres formed by these stacking forces between e.g. adjacent π-electrons in Fmoc groups create the stiff gels described.

The first and second peptide derivatives comprise gel-forming amino acids, referred to in the formula as GA.

In the hydrogels, X as defined generally can be (apart from phenylalanine) any naturally occurring amino acid. Suitable hydrogels can thus contain, as X, a neutral amino acid, a positively charged amino acid or a negatively charged amino acid. Overall, the invention thus provides a system for hydrogel preparation in which the resultant gels can have their hydrophobicity, charged nature and stiffness tuned according to the choice of components, including the choice of amino acids within the component first and second peptide derivatives.

In a set of embodiments of the invention, X is a neutral amino acid. In an example below, using a neutral amino acid rendered a gel having acceptable stiffness with the peptide concentrations tested, such as at concentrations of from 10mM to 30mM.

In a second set of embodiments of the invention, gels of the invention were prepared in which X is a charged amino acid, i.e. X is an amino acid selected from R, K, D and E. Gels based on peptides in which a charged amino acid is combined with F, as the GA component can exhibit improved stiffness compared with gels based on neutral amino acids. These gels were also more susceptible to modification of stiffness and other properties by including in the composition cations at varying concentrations. An advantage of these charged amino acid-containing gels is therefore the ability to further modify gel properties by addition of other compounds, molecules, peptides, peptide derivatives and especially cations into the gel composition, for example by addition of these components to a pre-gelation mixture.

In specific embodiments of the invention, X is an amino acid selected from Rand K. Gels based on using these positively charged amino acids showed high levels of stiffness even in the absence of additional components such as cations, yielding stiffness values of 10-20kPa and higher for peptide concentrations of 20mM - 30mM.

In further specific embodiments, X is an amino acid selected from D and E. Gels of the invention, based upon these negatively charged amino acids, yielded gels having acceptable stiffness, generally at similar levels to or of increased stiffness compared to a prior art hydrogel which is a combination of Fmoc-FF with Fmoc-S in a ratio of 1:1. Of note, however, is that gels of these specific embodiments, based on negatively charged amino acids, are susceptible to having their gel stiffness increased markedly by incorporation of cations into the composition, e.g. via addition of cation containing aqueous solutions to a pre-gelation mixture. As the individual stiffness requirements for the wide range of cell types that are to be cultured varies so greatly it is not practicable to provide a range of hydrogels that accommodate for each individual cell its optimum gel stiffness requirements. By using gels of the invention, especially those incorporating negatively charged amino acids, variation of the stiffness in the final gel can be achieved by variation of peptide and/or ion, especially cation, concentration, thus improving the usability and flexibility of the gel system of the invention.

The compositions of the invention can be provided in a dry form. By way of example, the composition can be in the form of a powder or lyophilized preparation. In one use water is added to the dry composition to yield a pre-gelation mixture, which in turn is combined with culture media to yield a gel. Alternatively, the dry form is combined in a single step with culture media to yield a mixture which itself spontaneously forms a gel. Dry formats of the composition can be prepared by subjecting an aqueous solution of the first and second peptides/derivatives to standard drying techniques. As one such example, a pre-gelation mixture is freeze-dried to yield a dry form of the invention.

As described widely herein, variation of gel components and concentrations can result in gels having a range of stiffness values. Suitably, the gel has a stiffness of from 100 Pa to 100 kPa. For most cell culture applications, peptide concentration and choice, according to the invention, yields a gel having a stiffness that is 500 Pa or greater, more preferably 1 kPa or greater, or preferably 2 kPa or greater. Tuning the properties of the gel prevents unacceptably high stiffness. Nevertheless, it is possible using the invention to produce gels having exceptionally high stiffness, for use in particular applications. Generally, the gels have a stiffness of up to about 70 kPa, preferably up to about 50 kPa, more preferably up to about 40 kPa.

In examples below, we describe gels of the invention using negatively charged amino acids having stiffness of from about 500 Pa to about 12 kPa. In other examples below, we describe particular gels of the invention based on positively charged amino acids, having stiffness in the range from about 3 kPa to about 24 kPa.

One advantage of the invention is the ability to produce stiff gels without having to use an excessively high peptide concentration. It is preferred that, in preparing compositions and gels of the invention the concentration of the peptide derivatives (treating for that purpose only the first and second peptide derivatives as a single derivative) in the final gel is from about 5mM to about 100mM, more preferably 10mM or higher, preferably up to about 70mM, or preferably up to about 50mM. In specific examples, gels based on peptide concentrations of approximately 20mM and 30mM both gave gels of high stiffness, thus without using excessively high peptide concentrations.

Compositions of the invention that are not provided as the final gel composition are provided dry or as pre-gelation mixtures. These can be marked to show the suitable amount of aqueous carrier to add to achieve the desired gel. In embodiments of the invention, therefore, the composition is provided in dry form or in the form of a pre-gelation mixture, for combination with a predetermined volume of aqueous carrier so that in the resultant gel the concentration of the peptide derivatives is from 5mM to 100mM, preferably 10mM to 50mM. Products in this form can hence be marked to indicate the gel properties obtained when the dry form or pre-dilation mixture is combined with the given carrier amounts. The products may also be marked to indicate how the gel properties will vary according to other components of the carrier such as ions, especially cations.

Particularly preferred embodiments are set out in examples below. These embodiments incorporate preferred selections for the various hydrogel composition components. Accordingly, in preferred embodiments of the invention, compositions are provided wherein one or more or all of:
(i) the ratio of first and second peptide derivatives is a ratio of from 2:3 to 3:2,
(ii) the ASL is Fmoc,
(iii) GA is phenylalanine, and
(iv) X is selected from S, R, K, D and E.

It is further preferred that the first and second peptide derivatives are substituted di-peptides, in which the first peptide derivatives comprise ASL-GA-GA-R₁ and the second peptide derivatives independently comprise ASL-GA-X-R₁. R₁ is independently selected from OH, O-protecting group, an amine, i.e. NH_{2,} or substituted amine, and ionised derivatives thereof.

Compositions of the invention have various applications, especially in cell culture. Accordingly, the invention further comprises compositions according to the invention further comprising cells, especially stem cells. Further compositions of the invention further comprise tissue, optionally derived from cells or stem cells cultured on the hydrogels of the invention.

Still further compositions provided herein include implants for use in animal, especially human, therapy comprising any composition of the invention as described above.

Still further provided by the invention is a method of culturing cells or tissue comprising combining cells or tissue with the composition of the invention, and culturing the same. Culturing may comprise growing the cells to increase the cell number (expanding the cells), differentiating the cells or both.

To help understanding of the invention, specific embodiments thereof will now be described by way of example and with reference to the accompanying drawings in which:
Fig. 1 is a photograph of gel samples of A) Fmoc-FF with Fmoc-FD, B) Fmoc-FF with Fmoc-FS, C) Fmoc-FF with Fmoc-FK , D) Fmoc-FF with Fmoc-FE, E) Fmoc-FF with Fmoc-FR and F) Fmoc-FF with Fmoc-S;
Fig. 2. is a graph of stiffness against gel concentration for the combinations described;
Fig. 3 is a series of photographs of live/dead staining after 1 day of culture of MSCs on A) Fmoc-FF with Fmoc-FS, B) Fmoc-FF with Fmoc-FS, C) Fmoc-FF with Fmoc-FK, D) Fmoc-FF with Fmoc-FD, E) Fmoc-FF with Fmoc-FE and F) Fmoc-FF with Fmoc-FR gels at 20mM concentration; and
Fig. 4 is a series of graphs of rheology results for peptide gel combinations over time for (a) 10mM gels, (b) 20mM gels, and (c) 30mM gels.

Combinations of peptides were investigated to determine particular combinations which produced hydrogels with properties (clarity, stiffness, longevity, physiological pH) which are advantageous for cell growth. Initial experiments concentrated on combinations of phenylalanine (F), with charged peptides and in particular aspartic acid (D), glutamic acid (E), lysine (K), arginine (R), and serine (S).

Combinations of the peptide derivatives Fmoc-FF with Fmoc-FD, Fmoc-FF with Fmoc-FE, and Fmoc-FF with Fmoc-FK were prepared as pre-gelation mixtures at approximately 1:1 ratios. The combinations of Fmoc-FF with Fmoc-FD and Fmoc-FF with Fmoc-FE and Fmoc-FF with Fmoc-FK all produced clear solutions.

In order to investigate the range of stiffness achievable from these combinations, as well as two additional combinations, namely Fmoc-FF with Fmoc-FR, and Fmoc-FF with Fmoc-FS, various different concentrations of these combinations were prepared as set out above, and all produced clear solutions, which then formed into clear hydrogels, with the exception of Fmoc-FF with Fmoc-FR. This produced a cloudy white solution and thus a cloudy hydrogel.

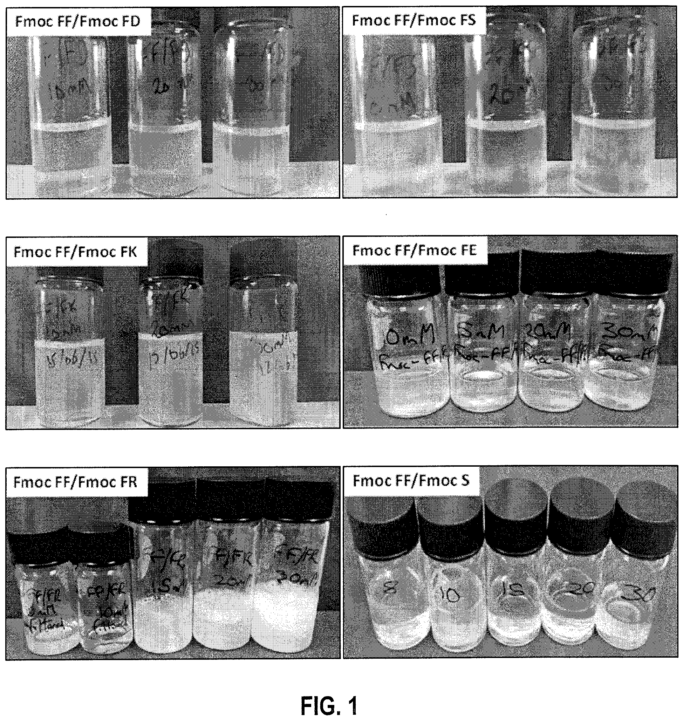

Fig. 1 shows the gel samples of A) Fmoc-FF with Fmoc-FD, B) Fmoc-FF with Fmoc-FS, C) Fmoc-FF with Fmoc-FK, all at three different concentrations, D) Fmoc-FF with Fmoc-FE at four different concentrations, E) Fmoc-FF with Fmoc-FR and F) Fmoc-FF with Fmoc-S at five different concentrations. The cloudy gels produced by the combination Fmoc-FF with Fmoc-FR can be seen.

A standard of a combination of Fmoc-FF with Fmoc-S was included in this experiment as a control.

In order to view cell growth on a hydrogel, in particular when using staining under a microscope, the provision of a clear hydrogel is advantageous. However, cloudiness does not affect cell growth, where the peptide or peptide combination is suitable, and thus for particular uses, for example where cells are growth for subsequent implantation, a cloudy hydrogel is not unsatisfactory.

The stiffness of all the combinations were assessed using rheology, as above, with the results set out in the Table 1 below (in which Fmoc-FF/S is not part of the present invention).

**Table 1**

| **Peptides** | **Stiffness (kPa)** | | | |
|---|---|---|---|---|
| **Concentration** | 10 mM | 15 mM | 20 mM | 30 mM |
| Fmoc-FF/S | 0.9 | 2.2 | 3.5 | 8.6 |
| Fmoc-FF/FE | 0.11 | 0.5 | 2.0 | 8.2 |
| Fmoc-FF/FD | 0.3 | 0.8 | 3.2 | 11.5 |
| Fmoc-FF/FS | 0.7 | 2.2 | 6.0 | 11.2 |
| Fmoc-FF/FR | 3.1 | 6.3 | 12.5 | 23.2 |
| Fmoc-FF/FK | 5.1 | 8.4 | 16.3 | 21.3 |

Different types of cell require a different stiffness in the scaffold for optimum growth. For example, brain cells require a relatively soft gel, while bone cells require a much stiffer gel. In order to support a variety of different cells it is desirable to be able to produce gels having a stiffness from 100 Pa to 50kPa. Thus the gels set out in Table 1 provided suitable stiffnesses for a variety of cells.

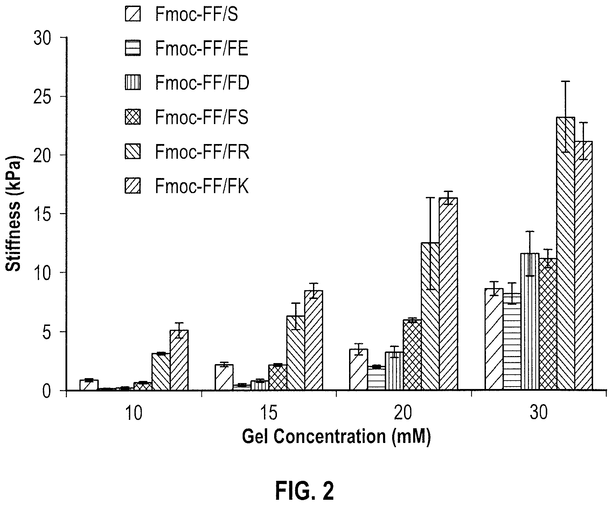

A graph of stiffness against gel concentration for the combinations described including the standard deviation is shown in Fig. 2.

This demonstrated that by altering the second peptide in the dipeptide sequence, a difference in scaffold strength is obtained when combined with an Fmoc-diphenylalanine peptide.

When comparing the combination of Fmoc-FF with Fmoc-S and Fmoc-FF with Fmoc-FS the results show an almost identical stiffness up to a gel concentration of 15 mM. For higher concentrations significantly higher stiffness is observed for Fmoc FF with Fmoc-FS gels. This is likely to be due to the increasing in π stacking of the additional phenylalanine ring being more effective at higher concentrations.

Combinations of Fmoc-FF with Fmoc-FD and Fmoc FF with Fmoc-FE show stiffnesses which were overall slightly below the stiffness range of the control, Fmoc-FF with Fmoc-S. Fmoc-FF with Fmoc-FK and Fmoc-FF with Fmoc-FR demonstrated a hugely increased stiffness at each concentration when compared to the control.

The positively charged groups on K and R seemed to enhance fibrous crosslinking producing significantly higher gel stiffnesses. This allows higher stiffness gels to be accessed without the need to increase fibre density.

The concentration of the peptides in the hydrogel is another factor which can affect cell growth due to the fibre density produced by the alignment of the peptides. Where the fibre density is too high, cell movement may be restricted and the gels become less amenable to cell growth. Concentration of the peptides is one factor which can affect the stiffness of the hydrogel, and thus a hydrogel suitable for promoting cell growth has a stiffness as set out above and yet not too high a concentration. Suitable concentrations of peptides will be up to a maximum of 100mM, and typically up to 40mM. Preferably, the hydrogel will have a peptide concentration of less than 30mM.

The experiments above describe the preparation and properties of hydrogels in various combinations and concentrations. The purpose of the hydrogels is to support cell growth and thus some of the combinations described above were also tested to determine cell viability of the hydrogels.

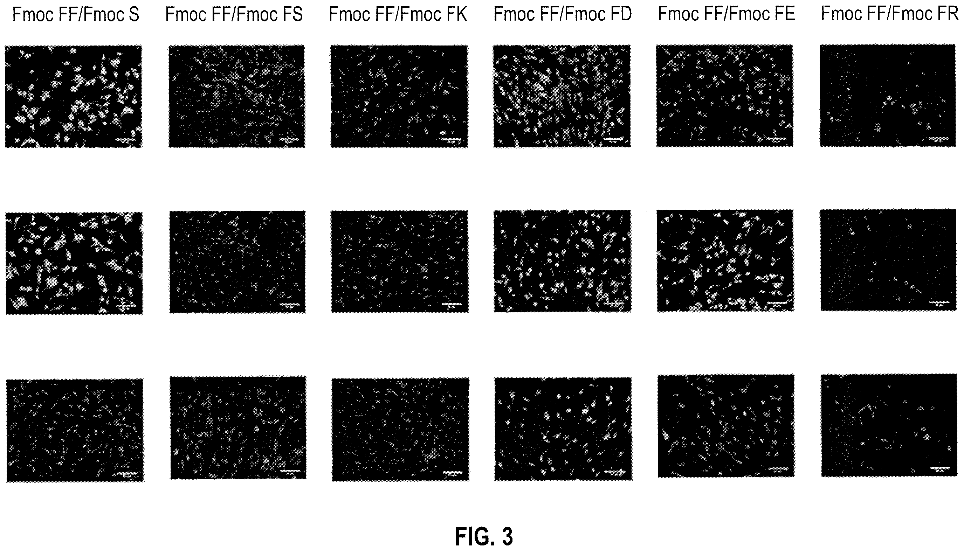

Hydrogels were formed as described above from combinations of Fmoc-FF with Fmoc-FD, Fmoc-FF with Fmoc-FE and Fmoc-FF with Fmoc-FK at a concentration of 20mM and seeded with cells, i.e. a 2D format. The cells were grown for 24 hours and a Live/Dead fluorescence assay with a confocal microscope was used to determine cell growth viability in the gels. Live/dead staining images are shown in Fig. 3.

The assay demonstrated good cell survival in all gels with minimal cell death. Thus the experiment confirmed that 20mM gels for the combinations described were suitable for supporting and growing varying cell populations.

Experiments were also carried out to note and ensure consistency of the gels produced. Combinations of Fmoc-FF with Fmoc-FS, Fmoc-FF with Fmoc-FK and Fmoc-FF with Fmoc-FD were chosen to give an overview of how the different charges incorporated in the sequence can affect the gel properties. Fmoc-FF with Fmoc-FK incorporates a positively charged amino acid (lysine), Fmoc-FF with Fmoc-FD incorporates a negatively charged amino acid (aspartic acid) and Fmoc-FF with Fmoc-FS incorporates a neutral but polar amino acid (serine).

A pre-gelation preparation method was formulated which was used for all peptide combinations and concentrations and demonstrated that gels could be prepared with a similar clear appearance for each combination.

The stiffness of each of these gels was then determined over a period of time to ensure that the gel had a suitable longevity for cell growth for different purposes.

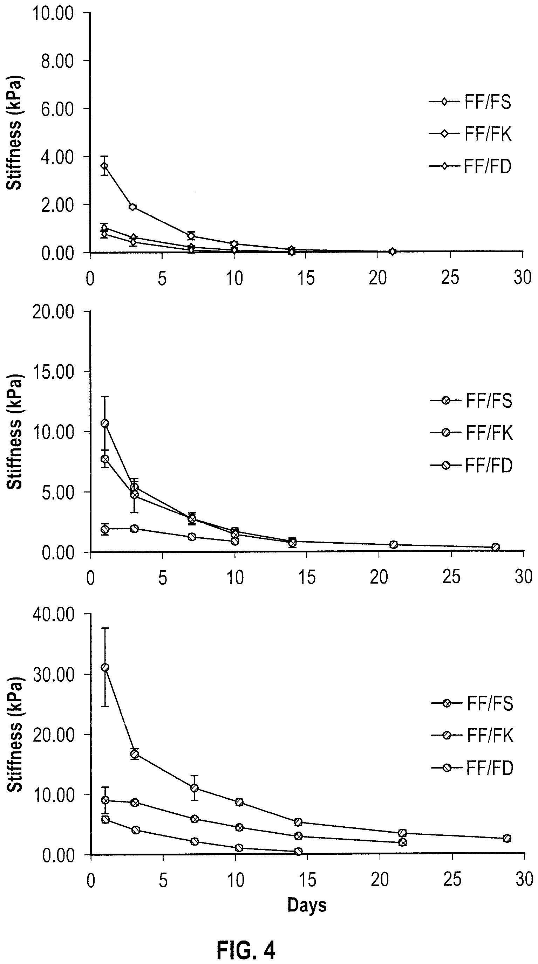

Pre-gelation mixtures for each of the peptide combinations were prepared at 10, 20 and 30 mM concentrations. Gels were prepared using DMEM cell culture media and the stiffness of the gels was assessed using rheology at day 1, 3, 7, 10, 14, 21 and 28. The graphs of Fig. 4 shows the stiffnesses obtained for each gel combination and concentration over time.

A general trend was that all gels showed a decrease in gel stiffness over time. Fmoc-FF with Fmoc-FK demonstrated the highest stiffness values over the time study, followed by Fmoc-FF with Fmoc-FS and the lowest stiffness values were seen from the Fmoc-FF with Fmoc-FD gels. However, the 10 mM Fmoc-FF with Fmoc-FS and Fmoc-FF with Fmoc-FD show similar values over a 7 day period.

Fmoc-FF with Fmoc-FK gels at higher concentrations, 20 and 30 mM, have shown to be stable up to 28 days in media with the lower concentration 10 mM gel viable up until the 21 day time point.

Fmoc-FF with Fmoc-FS gels have demonstrated that although there is a general decrease in stiffness over 10 days, the rate of decrease is less rapid than the Fmoc-FF with Fmoc-FK gels. This is highly beneficial as it enables gel consistency to be maintain over a longer period of time.

Thus all combinations provided hydrogels which are stable over a period of time useful for achieving cell growth and all can provide suitable scaffolds for cell growth depending on the type of cell to be supported and the duration of the requirement of the scaffold.

Thus these experiments have demonstrated using Fmoc-diphenylalanine combined with Fmoc-phenylalanine-X (X can be replaced by K, D, E, R and S) for producing hydrogels for supporting cell viability and growth at useful stiffness ranges without overly increasing fibre density. In addition, it has been demonstrated that such hydrogels subsist for at least 28 days, although with decreasing stiffness in the hydrogel.

The invention hence provides improved hydrogels and methods of making them and uses therefor.

## Claims

1. A hydrogel composition, comprising a mixture of first and second peptide derivatives in a ratio of from 1:4 to 4:1, wherein
the first peptide derivatives comprise ASL-GA-GA,
the second peptide derivatives comprise ASL-GA-X,
in which
ASL is an aromatic stacking ligand, which is Fmoc,
GA is the amino acid phenylalanine (F), and
X is an amino acid selected from neutral amino acid serine (S), positively charged amino acids arginine (R), and lysine (K), and negatively charged amino acids aspartic acid (D) and glutamic acid (E).

2. A composition according to any previous claim, in a dry form.

3. A composition according to claim 2, in the form of a powder or lyophilized preparation.

4. A composition according to any of claims 1 to 3, in the form of a gel or a pre-gelation preparation, which can form a gel upon addition of an aqueous carrier.

5. A composition according to claim 4, wherein the gel has a stiffness of from 100Pa to 100 kPa.

6. A composition according to any previous claim, in the form of a gel wherein the concentration of the peptide derivatives is from 5mM to 100mM, preferably 10mM to 50mM.

7. A composition according to any previous claim, wherein
i. the ratio of first and second peptide derivatives is from 2:3 to 3:2,
ii. the ASL is Fmoc,
iii. GA is phenylalanine, and
iv. X is selected from S, R, K, D and E.

8. A composition according to any previous claim, further comprising cells.

9. A composition according to claim 8, further comprising stem cells.

10. A composition according to any of claims 1 to 9, further comprising tissue.

11. An implant, comprising a composition according to claim 8, 9 or 10.

12. A method of culturing cells or tissue comprising combining the cells or tissue with a composition according to any of claims 1 to 7, and culturing the same.

## Patentansprüche

1. Hydrogelzusammensetzung, die ein Gemisch aus ersten und zweiten Peptidderivaten in einem Verhältnis von 1:4 bis 4:1 umfasst, wobei
das erste Peptidderivat ASL-GA-GA umfasst,
das zweite Peptidderivat ASL-GA-X umfasst,
wobei
ASL ein aromatischer Wechselwirkungsligand ist, der Fmoc ist,
GA die Aminosäure Phenylalanin (F) ist, und
X eine Aminosäure ist, die ausgewählt ist aus neutraler Aminosäure Serin (S), positiv geladenen Aminosäuren Arginin (R) und Lysin (K) und negativ geladenen Aminosäuren Asparaginsäure (D) und Glutaminsäure (E).

2. Zusammensetzung nach einem der vorstehenden Ansprüche in einer trockenen Form.

3. Zusammensetzung nach Anspruch 2 in Form eines Pulvers oder einer lyophilisierten Zubereitung.

4. Zusammensetzung nach einem der Ansprüche 1 bis 3 in Form eines Gels oder einer Vorgelierzubereitung, die bei Zusatz eines wässrigen Trägers ein Gel bilden kann.

5. Zusammensetzung nach Anspruch 4, wobei das Gel eine Steifheit von 100 Pa bis 100 kPa aufweist.

6. Zusammensetzung nach einem der vorstehenden Ansprüche in Form eines Gels, wobei die Konzentration der Peptidderivate zwischen 5 mM und 100 mM liegt, vorzugsweise zwischen 10 mM und 50 mM.

7. Zusammensetzung nach einem der vorstehenden Ansprüche, wobei
i. das Verhältnis des ersten zu dem zweiten Derivat zwischen 2:3 und 3:2 liegt;
ii. ASL Fmoc ist;
iii. GA Phenylalanin ist; und
iv. X ausgewählt ist aus S, R, K, D und E.

8. Zusammensetzung nach einem der vorstehenden Ansprüche, die ferner Zellen umfasst.

9. Zusammensetzung nach Anspruch 8, die ferner Stammzellen umfasst.

10. Zusammensetzung nach einem der Ansprüche 1 bis 9, die ferner Gewebe umfasst.

11. Implantat, das eine Zusammensetzung nach einem der Ansprüche 8, 9 oder 10 umfasst.

12. Verfahren zur Kultivierung von Zellen oder Gewebe, umfassend das Kombinieren der Zellen oder des Gewebes mit einer Zusammensetzung nach einem der Ansprüche 1 bis 7 und Kultivieren desselben.

## Revendications

1. Composition d'hydrogel, comprenant un mélange de premiers et seconds dérivés peptidiques dans un rapport de 1:4 à 4:1,
les premiers dérivés peptidiques comprenant ASL-GA-GA,
les seconds dérivés peptidiques comprenant ASL-GA-X,
ASL étant un ligand aromatique empilable, qui est Fmoc,
GA étant l'acide aminé phénylalanine (F), et
X étant un acide aminé choisi parmi l'acide aminé neutre sérine (S), les acides aminés chargés positivement arginine (R) et lysine (K), et les acides aminés chargés négativement acide aspartique (D) et acide glutamique (E).

2. Composition selon l'une quelconque des revendications précédentes, sous forme sèche.

3. Composition selon la revendication 2, sous la forme d'une poudre ou d'une préparation lyophilisée.

4. Composition selon l'une quelconque des revendications 1 à 3, sous la forme d'un gel ou d'une préparation pré-gel, qui peut former un gel par addition d'un véhicule aqueux.

5. Composition selon la revendication 4, le gel ayant une rigidité comprise entre 100 Pa et 100 kPa.

6. Composition selon l'une quelconque des revendications précédentes, sous la forme d'un gel, la concentration des dérivés peptidiques étant comprise entre 5 mM et 100 mM, de préférence entre 10 mM et 50 mM.

7. Composition selon l'une quelconque des revendications précédentes,
i. le rapport des premiers et seconds dérivés peptidiques étant de 2:3 à 3:2,
ii. ASL étant Fmoc,
iii. GA étant la phénylalanine, et
iv. X étant choisi parmi S, R, K, D et E.

8. Composition selon l'une quelconque des revendications précédentes, comprenant en outre des cellules.

9. Procédé selon la revendication 8, comprenant en outre des cellules-souches.

10. Composition selon l'une quelconque des revendications 1 à 9, comprenant en outre un tissu.

11. Implant, comprenant une composition selon la revendication 8, 9 ou 10.

12. Procédé de culture de cellules ou de tissu comprenant les étapes consistant à combiner des cellules ou du tissu avec une composition selon l'une quelconque des revendications 1 à 7, et à les cultiver.
